# EUROPEAN PATENT APPLICATION

(11) **EP 4 729 613 A1**
(43) Date of publication of application: **22.04.2026**
(21) Application number: 24823457.7
(22) Date of filing: 14.06.2024
(51) Int. Cl.: C12N 15/09, A61K 31/7088, A61K 35/12, A61K 35/76, A61K 48/00, A61P 27/02, C12N 5/10, C12N 15/63

(54) **ZINC FINGER NUCLEASE, VECTOR, AND METHOD FOR GENOME EDITING AND METHOD FOR VISUAL CELL PRODUCTION USING SAID NUCLEASE AND VECTOR**

(30) Priority: 16.06.2023 JP 2023098875
(71) Applicant: VCGT Inc., Kobe-shi, Hyogo, 650-0047 (JP); Hiroshima University, Higashi-Hiroshima-shi Hiroshima 739-8511 (JP)
(72) Inventor: ONISHI, Akishi, Kobe-shi, Hyogo 650-0047 (JP); YASUDA, Kazushi, Kobe-shi, Hyogo 650-0047 (JP); ISHIMARU, Aiko, Kobe-shi, Hyogo 650-0047 (JP); SAKUMA, Tetsushi, Hiroshima-shi, Hiroshima 739-8511 (JP); YAMAMOTO, Takashi, Hiroshima-shi, Hiroshima 739-8511 (JP); NOMURA, Wataru, Hiroshima-shi, Hiroshima 739-8511 (JP)
(74) Representative: Henderson, Helen Lee
(86) International application number: PCT/JP2024/021600
(87) International publication number: WO 2024/257844

(57) **Abstract**

The present invention provides, as a technology useful for genome editing of the rhodopsin (RHO) gene locus by using zinc-finger nucleases (ZFNs), a combination of a pair of ZFNs each having a zinc-finger array recognizing a specific nucleotide sequence.

## Description

### Technical Field

The present disclosure relates to a zinc-finger nuclease, a vector, and a genome editing method and cell production method using them. The present disclosure relates more specifically to, for example, a zinc-finger nuclease for genome editing at the rhodopsin (RHO) gene locus.

### Background Art

The human rhodopsin protein is a light-sensitive G protein-coupled receptor (GPCR) specific to rod photoreceptors, and consists of approximately 348 amino acids. The rhodopsin (RHO) gene locus, which encodes the rhodopsin protein, includes five exons and is approximately 6.7 kb long (chr3: 129,528,639-129,535,344) including intron regions. Mutations in the rhodopsin gene cause retinitis pigmentosa, and about 110 types of gene mutations have been reported so far. Patent Literature 1 proposes a technology for treating retinitis pigmentosa, which is caused by mutations in the rhodopsin gene, by genome editing using designer nucleases such as TALE nuclease (TALEN), zinc-finger nuclease (ZFN), and Cas nuclease.

The ZFN consists of a nuclease domain, namely FokI-ND, a nucleotide-binding domain, namely a zinc-finger protein (ZFP), and a linker connecting them. Patent Literature 2 discloses a ZFN containing a nuclease domain called nuclease domain 1 (ND1) or nuclease domain 2 (ND2) instead of FokI-ND. ND1 is the nuclease domain derived from *Bacillus* sp. SGD-V-76, and ND2 is the nuclease domain derived from *Clostridium botulinum.* ND1 and ND2 are considered to be superior to the conventional FokI-ND in terms of functionalities such as cleavage activity, specificity, and target sequence selectivity.

### Citation List

### Patent Literature

Patent Literature 1: WO 2021/010303
Patent Literature 2: WO 2020/045281

### Non Patent Literature

Non Patent Literature 1: "Standardized reagents and protocols for engineering zinc-finger nucleases by modular assembly", Wright et al., Nature Protocols, 2006, Vol. 1, No. 3, p. 1637-52.

### Summary of Invention

### Technical Problem

The main purpose of the present disclosure is to provide a technology useful for genome editing of the RHO gene locus while using a ZFN.

### Solution to Problem

To solve the above problem, the present disclosure provides the following [1] to [12].
[1] A combination of a pair of zinc-finger nucleases (ZFNs) for genome editing of a rhodopsin (RHO) gene locus, the combination selected from the following group:
   (1) a combination of a ZFN with a zinc-finger array recognizing a nucleotide sequence of SEQ ID NO: 1 and a ZFN with a zinc-finger array recognizing a nucleotide sequence of SEQ ID NO: 2;
   (2) a combination of a ZFN with a zinc-finger array recognizing a nucleotide sequence of SEQ ID NO: 5 and a ZFN with a zinc-finger array recognizing a nucleotide sequence of SEQ ID NO: 6;
   (3) a combination of a ZFN with a zinc-finger array recognizing a nucleotide sequence of SEQ ID NO: 7 and a ZFN with a zinc-finger array recognizing a nucleotide sequence of SEQ ID NO: 8;
   (4) a combination of a ZFN with a zinc-finger array recognizing a nucleotide sequence of SEQ ID NO: 9 and a ZFN with a zinc-finger array recognizing a nucleotide sequence of SEQ ID NO: 10;
   (5) a combination of a ZFN with a zinc-finger array recognizing a nucleotide sequence of SEQ ID NO: 11 and a ZFN with a zinc-finger array recognizing a nucleotide sequence of SEQ ID NO: 12;
   (6) a combination of a ZFN with a zinc-finger array recognizing a nucleotide sequence of SEQ ID NO: 13 and a ZFN with a zinc-finger array recognizing a nucleotide sequence of SEQ ID NO: 14;
   (7) a combination of a ZFN with a zinc-finger array recognizing a nucleotide sequence of SEQ ID NO: 15 and a ZFN with a zinc-finger array recognizing a nucleotide sequence of SEQ ID NO: 16;
   (8) a combination of a ZFN with a zinc-finger array recognizing a nucleotide sequence of SEQ ID NO: 31 and a ZFN with a zinc-finger array recognizing a nucleotide sequence of SEQ ID NO: 32;
   (9) a combination of a ZFN with a zinc-finger array recognizing a nucleotide sequence of SEQ ID NO: 35 and a ZFN with a zinc-finger array recognizing a nucleotide sequence of SEQ ID NO: 36;
   (10) a combination of a ZFN with a zinc-finger array recognizing a nucleotide sequence of SEQ ID NO: 37 and a ZFN with a zinc-finger array recognizing a nucleotide sequence of SEQ ID NO: 6;
   (11) a combination of a ZFN with a zinc-finger array recognizing a nucleotide sequence of SEQ ID NO: 38 and a ZFN with a zinc-finger array recognizing a nucleotide sequence of SEQ ID NO: 8;
   (12) a combination of a ZFN with a zinc-finger array recognizing a nucleotide sequence of SEQ ID NO: 7 and a ZFN with a zinc-finger array recognizing a nucleotide sequence of SEQ ID NO: 10;
   (13) a combination of a ZFN with a zinc-finger array recognizing a nucleotide sequence of SEQ ID NO: 11 and a ZFN with a zinc-finger array recognizing a nucleotide sequence of SEQ ID NO: 14;
   (14) a combination of a ZFN with a zinc-finger array recognizing a nucleotide sequence of SEQ ID NO: 15 and a ZFN with a zinc-finger array recognizing a nucleotide sequence of SEQ ID NO: 18;
   (15) a combination of a ZFN with a zinc-finger array recognizing a nucleotide sequence of SEQ ID NO: 45 and a ZFN with a zinc-finger array recognizing a nucleotide sequence of SEQ ID NO: 32; and
   (16) a combination of a ZFN with a zinc-finger array recognizing a nucleotide sequence of SEQ ID NO: 47 and a ZFN with a zinc-finger array recognizing a nucleotide sequence of SEQ ID NO: 36.
[2] The combination according to [1], wherein the combination is selected from the following group:
   (2) a combination of a ZFN with a zinc-finger array recognizing a nucleotide sequence of SEQ ID NO: 5 and a ZFN with a zinc-finger array recognizing a nucleotide sequence of SEQ ID NO: 6;
   (4) a combination of a ZFN with a zinc-finger array recognizing a nucleotide sequence of SEQ ID NO: 9 and a ZFN with a zinc-finger array recognizing a nucleotide sequence of SEQ ID NO: 10;
   (5) a combination of a ZFN with a zinc-finger array recognizing a nucleotide sequence of SEQ ID NO: 11 and a ZFN with a zinc-finger array recognizing a nucleotide sequence of SEQ ID NO: 12;
   (10) a combination of a ZFN with a zinc-finger array recognizing a nucleotide sequence of SEQ ID NO: 37 and a ZFN with a zinc-finger array recognizing a nucleotide sequence of SEQ ID NO: 6;
   (12) a combination of a ZFN with a zinc-finger array recognizing a nucleotide sequence of SEQ ID NO: 7 and a ZFN with a zinc-finger array recognizing a nucleotide sequence of SEQ ID NO: 10;
   (13) a combination of a ZFN with a zinc-finger array recognizing a nucleotide sequence of SEQ ID NO: 11 and a ZFN with a zinc-finger array recognizing a nucleotide sequence of SEQ ID NO: 14; and
   (14) a combination of a ZFN with a zinc-finger array recognizing a nucleotide sequence of SEQ ID NO: 15 and a ZFN with a zinc-finger array recognizing a nucleotide sequence of SEQ ID NO: 18.
[3] The combination according to [1] or [2], wherein a spacer between the nucleotide sequences recognized by the two zinc-finger arrays is 5 bps or 6 bps.
[4] The combination according to any one of [1] to [3], wherein a nuclease domain of each ZFN is ND1.
[5] The combination according to [4], wherein the nuclease domain of the ZFN comprises
   an amino acid sequence at positions 391 to 585 of SEQ ID NO: 52,
   an amino acid sequence of SEQ ID NO: 57, or
   an amino acid sequence of SEQ ID NO: 58.
[6] The combination according to any one of [1] to [4], wherein the RHO gene locus is a human RHO gene locus.
[7] A vector comprising a nucleotide sequence encoding any one of the pairs of ZFNs according to [1] to [6].
[8] The vector according to [7], wherein the vector is a bidirectional expression vector and
   the vector comprises a nucleotide sequence encoding a sense strand ZFN and a promoter sequence controlling transcription of the nucleotide sequence, and
   a nucleotide sequence encoding an antisense strand ZFN and a promoter sequence controlling transcription of the nucleotide sequence,
   provided that the two promoter sequences are arranged consecutively without a spacer sequence therebetween.
[9] The vector according to [8], wherein the two promoters are CMV promoter and human EF1a (Elongation Factor alpha-1) promoter.
[10] A therapeutic agent for a disease derived from an RHO gene, comprising the vector according to any one of [7] to [9].
[11] A method for genome editing of an RHO gene locus, comprising, as a procedure, introducing the vector according to any one of [7] to [9] into a photoreceptor cell *in vivo* or *in vitro,* preferably *in vitro.*
[12] A method for producing a photoreceptor cell having an RHO gene genome-edited, comprising, as a procedure, introducing the vector according to any one of [7] to [9] into a photoreceptor cell *in vivo* or *in vitro,* preferably *in vitro.*

### Advantageous Effects of Invention

The present disclosure provides a technology useful for genome editing of the RHO gene locus while using ZFNs.

### Brief Description of Drawings

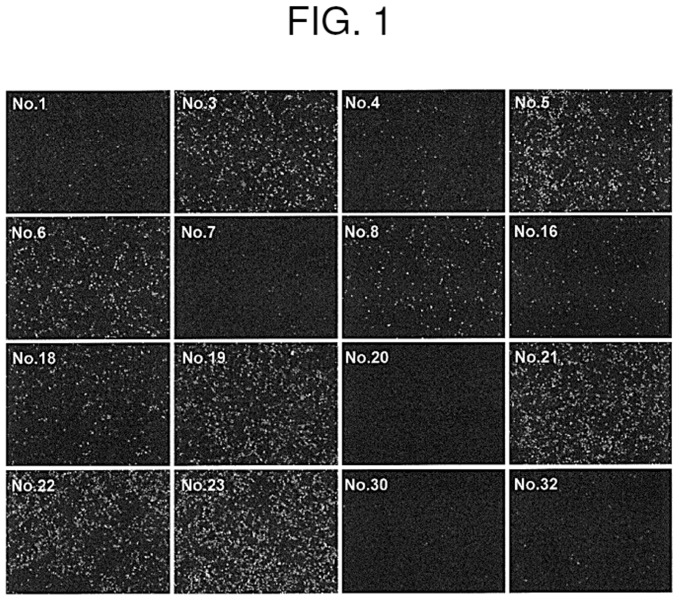

[Fig. 1] Fig. 1 is EGFP fluorescence images of HEK293 cells transfected with a ZF-ND1 expression plasmid and an EGFP fragment expression plasmid containing a candidate ZF-binding target sequence. Fluorescence of EGFP indicates cleavage of a candidate ZF-binding target sequence by ZF-ND1.

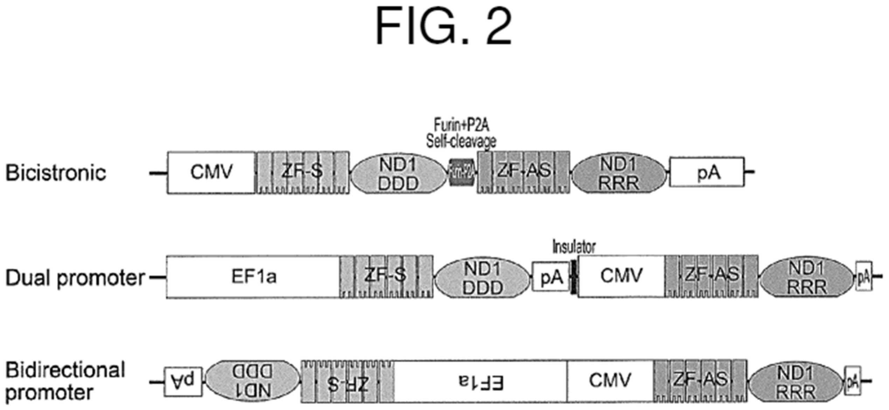

[Fig. 2] Fig. 2 shows the configuration of Bicistronic, Dual promoter, and Bidirectional promoter plasmid vectors.

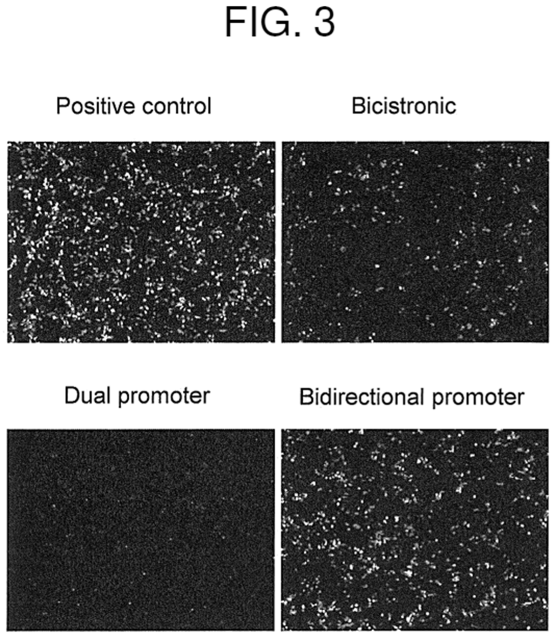

[Fig. 3] Fig. 3 is EGFP fluorescence images of HEK293 cells transfected with a Bicistronic, Dual promoter, or Bidirectional promoter expression plasmid and an EGFP fragment expression plasmid containing a candidate ZF-binding target sequence. Fluorescence of EGFP indicates cleavage of a candidate ZF-binding target sequence by ZF-ND1. As a positive control, ZF-ND1 expression plasmids (5461S and 5562AS) were introduced.

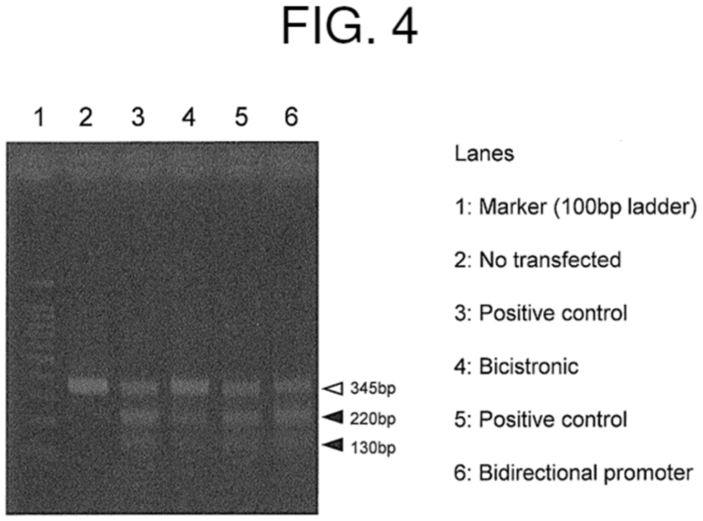

[Fig. 4] Fig. 4 shows the results of Cel-I analysis of the target RHO sequence in HEK293 cells transfected with a Bicistronic or Bidirectional promoter plasmid. The bands at 220 bp and 130 bp indicate InDel (Insertion and Deletion). ZF-ND1 expression plasmids (5461S and 5562AS) were used as a positive control.

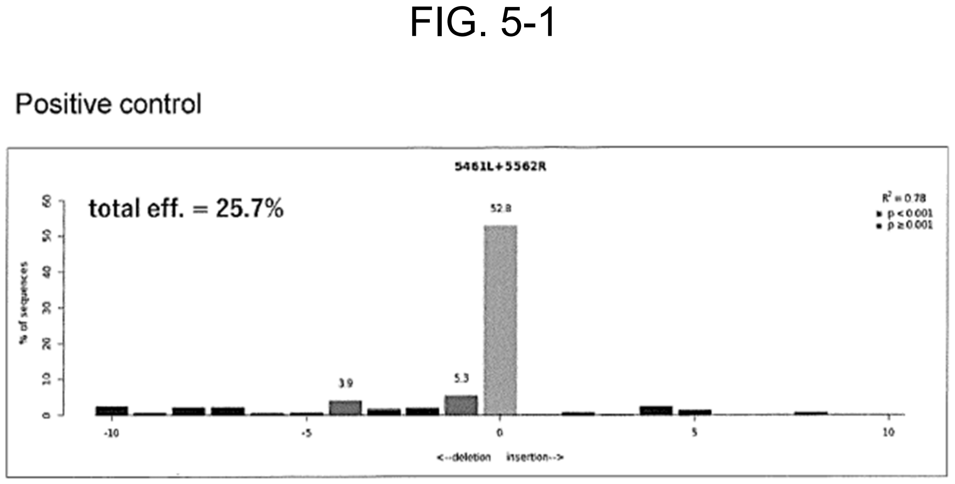

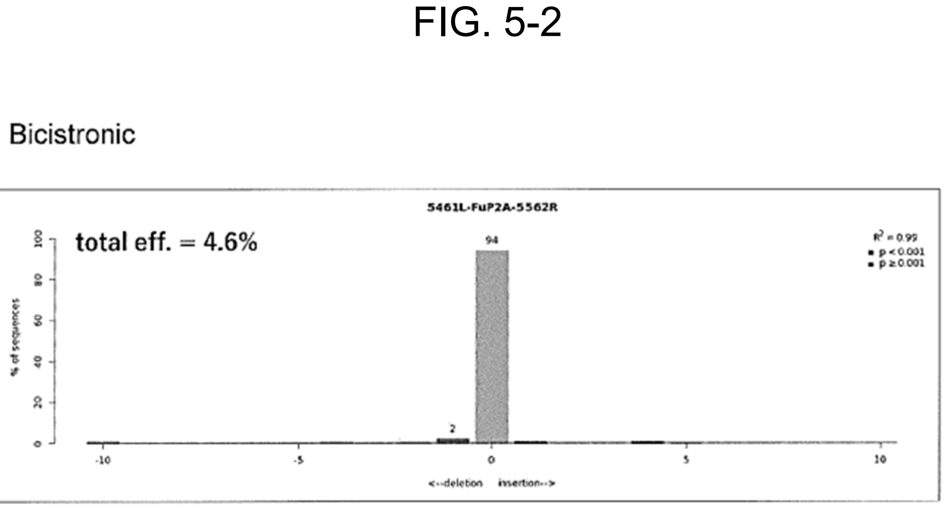

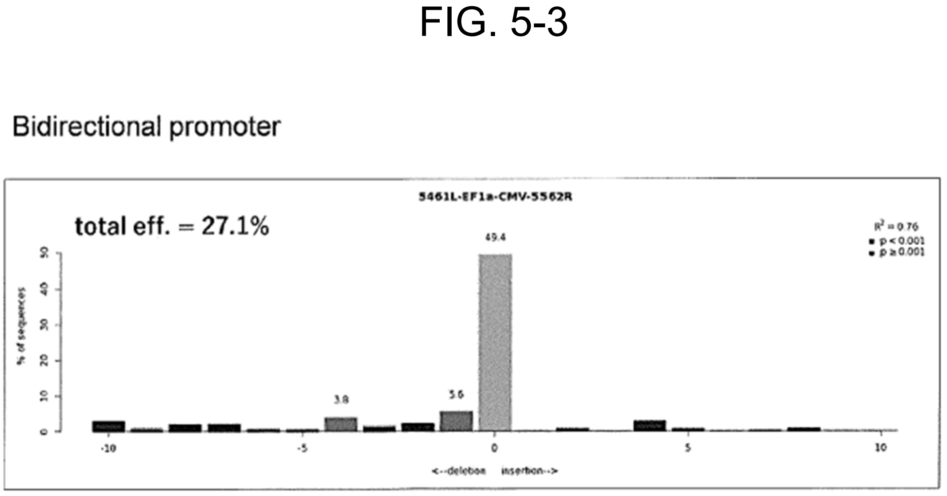

[Fig. 5] Fig. 5 shows the results of Tide (Tracking of Indels by DEcomposition) analysis of the target RHO sequence in HEK293 cells transfected with a Bicistronic or Bidirectional promoter plasmid. The 0 on the x-axis of the histogram indicates the same sequence as WT (no InDel), the positive direction indicates the number of Insertion nucleotides, the negative direction indicates the number of Deletion nucleotides, and the y-axis indicates their frequency (percentage). The "Total eff" indicates the sum (%) of InDel frequencies. ZF-ND1 expression plasmids (5461S and 5562AS) were used as a positive control.

### Description of Embodiments

### 1. Zinc-finger nuclease (ZFN)

The ZFNs of the present disclosure can be used for genome editing of the rhodopsin (RHO) gene locus and are a combination of ZFNs each having a zinc-finger array that recognizes a specific nucleotide sequence. The Zinc-finger array is composed of an array having multiple zinc-fingers (ZFs) each recognizing a three-nucleotide sequence. The zinc-finger array may contain two or more ZFs, e.g., 3-9, preferably 4-8, more preferably 5-7, or typically 6 ZFs.

The relationship between the amino acid sequence of ZF and the three-nucleotide sequence recognized by ZF is well-known, and the amino acid sequence of ZF can be used, for example, as described in Supplemental Table 1 of Non-Patent Literature 1, depending on the three-nucleotide sequence to be recognized. Since there are multiple ZF amino acid sequences that recognize the same three-nucleotide sequence, these ZFs that recognize the same three-nucleotide sequence can be used interchangeably.

The ZFNs of the present disclosure have zinc-finger arrays selected from the following combinations (see Table 1 below):
(1) a combination of a ZFN with a zinc-finger array recognizing a nucleotide sequence of SEQ ID NO: 1 and a ZFN with a zinc-finger array recognizing a nucleotide sequence of SEQ ID NO: 2;
(2) a combination of a ZFN with a zinc-finger array recognizing a nucleotide sequence of SEQ ID NO: 5 and a ZFN with a zinc-finger array recognizing a nucleotide sequence of SEQ ID NO: 6;
(3) a combination of a ZFN with a zinc-finger array recognizing a nucleotide sequence of SEQ ID NO: 7 and a ZFN with a zinc-finger array recognizing a nucleotide sequence of SEQ ID NO: 8;
(4) a combination of a ZFN with a zinc-finger array recognizing a nucleotide sequence of SEQ ID NO: 9 and a ZFN with a zinc-finger array recognizing a nucleotide sequence of SEQ ID NO: 10;
(5) a combination of a ZFN with a zinc-finger array recognizing a nucleotide sequence of SEQ ID NO: 11 and a ZFN with a zinc-finger array recognizing a nucleotide sequence of SEQ ID NO: 12;
(6) a combination of a ZFN with a zinc-finger array recognizing a nucleotide sequence of SEQ ID NO: 13 and a ZFN with a zinc-finger array recognizing a nucleotide sequence of SEQ ID NO: 14;
(7) a combination of a ZFN with a zinc-finger array recognizing a nucleotide sequence of SEQ ID NO: 15 and a ZFN with a zinc-finger array recognizing a nucleotide sequence of SEQ ID NO: 16;
(8) a combination of a ZFN with a zinc-finger array recognizing a nucleotide sequence of SEQ ID NO: 31 and a ZFN with a zinc-finger array recognizing a nucleotide sequence of SEQ ID NO: 32;
(9) a combination of a ZFN with a zinc-finger array recognizing a nucleotide sequence of SEQ ID NO: 35 and a ZFN with a zinc-finger array recognizing a nucleotide sequence of SEQ ID NO: 36;
(10) a combination of a ZFN with a zinc-finger array recognizing a nucleotide sequence of SEQ ID NO: 37 and a ZFN with a zinc-finger array recognizing a nucleotide sequence of SEQ ID NO: 6;
(11) a combination of a ZFN with a zinc-finger array recognizing a nucleotide sequence of SEQ ID NO: 38 and a ZFN with a zinc-finger array recognizing a nucleotide sequence of SEQ ID NO: 8;
(12) a combination of a ZFN with a zinc-finger array recognizing a nucleotide sequence of SEQ ID NO: 7 and a ZFN with a zinc-finger array recognizing a nucleotide sequence of SEQ ID NO: 10;
(13) a combination of a ZFN with a zinc-finger array recognizing a nucleotide sequence of SEQ ID NO: 11 and a ZFN with a zinc-finger array recognizing a nucleotide sequence of SEQ ID NO: 14; and
(14) a combination of a ZFN with a zinc-finger array recognizing a nucleotide sequence of SEQ ID NO: 15 and a ZFN with a zinc-finger array recognizing a nucleotide sequence of SEQ ID NO: 18.
(15) a combination of a ZFN with a zinc-finger array recognizing a nucleotide sequence of SEQ ID NO: 45 and a ZFN with a zinc-finger array recognizing a nucleotide sequence of SEQ ID NO: 32; and
(16) a combination of a ZFN with a zinc-finger array recognizing a nucleotide sequence of SEQ ID NO: 47 and a ZFN with a zinc-finger array recognizing a nucleotide sequence of SEQ ID NO: 36.

Table 1 shows the recognition sequence of each ZFN zinc-finger array, their SEQ ID NO., and amino acid sequence of the zinc-finger array.

For example, a pair of ZFNs in (1) above is a combination of a sense strand ZFN 4350S having a zinc-finger array recognizing the nucleotide sequence of SEQ ID NO: 1 (ATTCTTGGGTGGGAGCAG) and an antisense strand ZFN 4350AS having a zinc-finger array recognizing the nucleotide sequence of SEQ ID NO: 2 (AAGGCCTGAGCTCAGCCA). ZFN 4350S consists of the amino acid sequence of SEQ ID NO: 59, and ZFN 4350AS consists of the amino acid sequence of SEQ ID NO: 60.

For example, the ZFN 4350S zinc-finger array has an amino acid sequence in which ZF91, ZF62, ZF106, ZF58, ZF103, and ZF88 (see Non-Patent Literature 1, Supplemental Table 1) that recognize CAG, GAG, TGG, GGG, CTT, and ATT, respectively, are linked from the N-terminal end.

The ZFNs of the present disclosure are more preferably a combination of any of (2), (4), (5), (10), (12), (13), or (14) above because their cleavage activity is high.

The ZFNs of the present disclosure recognize and bind nucleotide sequences upstream of the start codon of exon 1 of the RHO gene (5'UTR at the RHO gene locus) in the RHO gene locus by zinc-finger arrays, resulting in a DNA double-strand break (DSB) by the nuclease domain.

The length of the spacer between the nucleotide sequences recognized by the two zinc-finger arrays of the ZFNs of the present disclosure is not particularly limited, but is, for example, 1-20 bp, preferably 3-8 bp, or 5 bps or 6 bps. The nucleotide sequence of the spacer is uniquely defined as the nucleotide sequence between the nucleotide sequence recognized by one zinc-finger array and the nucleotide sequence recognized by the other one. The ZFNs cleaves a DNA double-strand in the spacer sequence.

For example, a pair of ZFNs in (1) above cleaves a DNA double-strand in a spacer sequence (CGCAGC) between the nucleotide sequence of SEQ ID NO: 1 (ATTCTTGGGTGGGAGCAG), which is the binding sequence of the sense strand ZFN, and the nucleotide sequence of SEQ ID NO: 2 (AAGGCCTGAGCTCAGCCA), which is the binding sequence of the antisense strand ZFN.

The nuclease domain of the ZFN of the present disclosure is not particularly limited and may be FokI-ND, ND1, or ND2 described in Patent Literature 2, or their modified forms. The amino acid sequence of the full-length protein containing ND1 (from *Bacillus* sp. SGD-V-76) is set forth in SEQ ID NO: 52 and its nucleotide sequence in SEQ ID NO: 53. The amino acid sequence of the full-length protein containing ND2 (from *Clostridium botulinum*) is set forth in SEQ ID NO: 54 and its nucleotide sequence in SEQ ID NO: 55. ND1 is typically a partial peptide corresponding to positions 391-585 of SEQ ID NO: 52, and ND2 is typically a partial peptide corresponding to positions 389-579 of SEQ ID NO: 54. The nuclease domain should be ND1.

Modified forms of ND1 and ND2 may each contain an amino acid sequence in which one or several amino acid residues are substituted, deleted, inserted, or added in the amino acid sequence at positions 391 to 585 of SEQ ID NO: 52 or 389 to 579 of SEQ ID NO: 54, and may be a polypeptide having nuclease activity. Here, the "one or several" means, for example, 1 to 10, preferably 1 to 6, e.g., 1, 2, 3, 4, or 5.

Modified forms of ND1 and ND2 may contain an amino acid sequence having at least 70%, at least 75%, at least 80%, at least 85%, at least 90%, at least 95%, or at least 98% sequence identity to the amino acid sequence at positions 391 to 585 of SEQ ID NO: 52 or 389 to 579 of SEQ ID NO: 54, and may be a polypeptide having nuclease activity. Here, the "sequence identity" of an amino acid sequence is determined by comparing two sequences aligned such that the identity between sequences is maximum. How to calculate the sequence identity percentage (%) is well-known to those skilled in the art. As an algorithm to obtain the best alignment and sequence identity, any of the algorithms (e.g., BLAST algorithm, FASTA algorithm) known to those skilled in the art should be used. The sequence identity of an amino acid sequence is determined using sequence analysis software such as BLASTP or FASTA.

Specific examples of the modified forms of ND1 and ND2 include a modified form (DDD-type variant) having aspartic acid (D483, D487, and D496) at positions corresponding to positions 483, 487, and 496 in the amino acid sequence of FokI, or an modified form (RRR-type variant) having arginine (R483, R487, and R537) at positions corresponding to positions 483, 487, and 537 in the amino acid sequence of FokI. The DDD-type variant of ND1 (ND1DDD) has an amino acid sequence (SEQ ID NO: 57) in which amino acids 103 and 113 are replaced with aspartic acid in the partial peptide corresponding to positions 391-585 of SEQ ID NO: 52. In addition, the RRR-type variant of ND1 (ND1RRR) has an amino acid sequence (SEQ ID NO: 58) in which amino acids 100 and 154 are replaced with arginine in the partial peptide corresponding to positions 391-585 of SEQ ID NO: 52.

The nuclease domain of ZFN of the present disclosure may be a polypeptide containing modified and/or non-natural amino acids. Examples of the modified amino acid include, but are not limited to, methylation, esterification, amidation, acetylation, alkylation, and halogenation. The modified and non-natural amino acids can be introduced by known procedures.

The nuclease domain and the zinc-finger protein (ZFP) containing a zinc-finger array may be linked directly or via a linker. The linker may, for example, comprise two or more amino acid residues and examples of the length include, but are not particularly limited to, 2 to 20 amino acids, e.g., 2, 3, 4, 5, 6, 7, 8, 9, 10, 11, 12, 13, 14, 14, 16, 17, 18, 19, or 20 amino acids. The type of linker is also not particularly limited, and examples include, but are not particularly limited to, TGGS (SEQ ID NO: 56). The presence or absence of the linker and the length and type of the linker are appropriately selected by a person skilled in the art in consideration of the type of nucleotide binding domain and other factors.

The ZFN of the present disclosure may have another effector domain instead of the nuclease domain. Examples of such an effector domain include Cytosine base editor (UGI, APOBEC), Adenine base editor (TadA), transcriptional activator (VP16)/repressor (SID), epigenomic editor (DNMT3A, TET1), imaging protein (Dye, fluorescent protein), transposase (PiggyBac), or recombinase (Flippase).

The ZFNs of the present disclosure can be produced *in vitro* or *in vivo* by methods known in the art. Examples include an artificial synthesis method based on amino acid sequence information, or a method in which a nucleic acid encoding each ZFN is artificially synthesized, inserted into a suitable expression vector, and introduced in a suitable host cell to express the ZFNs in the cell.

### 2. Vector

The vector according to the present disclosure contains nucleotide sequences encoding a pair of ZFNs described above. In one embodiment, the vector of the present disclosure is a bidirectional expression vector, in which the sense strand ZFN and antisense strand ZFN of a pair of ZFNs are expressed under the control of separate promoters, and these two promoters are arranged in opposite directions. More specifically, the bidirectional expression vector of the present disclosure contains a nucleotide sequence encoding a sense strand ZFN and a promoter sequence that controls transcription of the nucleotide sequence, and a nucleotide sequence encoding an antisense strand ZFN and a promoter sequence that controls transcription of the nucleotide sequence. It is preferred that the two promoter sequences are arranged consecutively without a spacer sequence between them.

In the vector of the present disclosure, conventionally known promoters can be used for the two promoters. Examples of the promoter that can be used include CMV promoter, human EF1a (Elongation Factor alpha-1) promoter, and PGK (phosphoglycerate kinase) promoter. The two promoters are preferably CMV promoter and human EF1a.

In addition to the nucleotide sequence encoding each ZFN and their promoter sequence, the vector of the present disclosure may contain elements provided by conventional gene expression vectors. Examples of such an element include a poly(A) addition sequence (e.g., SV40 p(A), HGH p(A), rabbit globin p(A)).

The vector is not particularly limited and can be selected from vectors used in the art. Specific examples of the vector include phage vectors, plasmid vectors, viral vectors, retroviral vectors, chromosomal vectors, episomal vectors, virus-derived vectors (e.g., bacterial plasmids, bacteriophages, yeast episomes), yeast chromosomal elements, viruses (e.g., baculovirus, papovavirus, vaccinia virus, adenovirus, adeno-associated virus, fowlpox virus, pseudorabies virus, herpesvirus, lentivirus, retrovirus), and vectors derived from combinations thereof (e.g., cosmids, phagemids). Among the above, plasmid vectors are preferred in terms of versatility. From the viewpoint of advanced clinical application, viral vectors are preferred, and adeno-associated virus vectors (AAV vectors) are more preferred.

### 3. Genome editing method, etc.

The present disclosure also provides a method for genome editing of the RHO gene locus of a photoreceptor cell, including, as a procedure, introducing the above-described vector into a photoreceptor cell *in vivo* or *in vitro,* preferably in vitro, and a method for producing a genome-edited photoreceptor cell. The ZFN-based genome editing system is composed solely of proteins, unlike the Cas nuclease-based genome editing system, which involves ubiquitous expression of gRNA. Therefore, it is easy to realize tissue-specific genome editing by using tissue-specific promoters, drug-responsive promoters or the like in the ZFN-based genome editing system.

Introduction of the vector into a photoreceptor cell can be performed by conventionally known methods. Examples of the introduction method include, but are not limited to, electroporation, particle gun, microinjection, lipofection, and protein transduction.

Depending on the purpose of genome editing, a donor DNA may be introduced into the cell together with the vector. The donor DNA may be, for example, a donor DNA encoding a normal RHO gene. The donor DNA can be configured to introduce a normal RHO gene into the gene locus by using a repair mechanism such as nonhomologous endjoining (NHEJ) or homologous recombination (HR) at the cleavage site of DNA double-strand break by ZFNs.

In the case of genome editing without any donor DNA, the cleavage site is repaired mainly by NHEJ. Because NHEJ is error-prone, at least one nucleotide deletion, insertion, or substitution, or a combination thereof, can occur during repair of the cleavage. In this way, the RHO gene locus is modified at the cleavage site.

Photoreceptor cells may be of human or non-human animal origin. Examples of the non-human animals include even-toed ungulates (e.g., cattle, boars, pigs, sheep, goats), odd-toed ungulates (e.g., horses), rodents (e.g., mice, rats, hamsters, squirrels), hares (e.g., rabbits), and carnivores (e.g., dogs, cats, ferrets).

### 4. Therapeutic agent

The present disclosure also provides a therapeutic agent for a disease derived from the RHO gene, comprising the vector described above. The therapeutic agent may contain a donor DNA encoding the above-described normal RHO gene.

In addition, the genome-edited photoreceptor cell obtained by the above-mentioned genome editing method can be formulated as a cell pharmaceutical. Such a cell pharmaceutical may also be a therapeutic agent for a disease derived from the RHO gene.

The therapeutic agent may be prepared according to a common procedure. Specifically, the above-mentioned vector and optionally the above-mentioned donor DNA can be formulated by mixing them with pharmaceutical additives, or by mixing genome-edited photoreceptor cells with pharmaceutical additives.

The pharmaceutical additives are intended for substances other than the active ingredient contained in the preparation. The pharmaceutical additives are included in the preparation to facilitate its formulation, stabilize its quality, or enhance its usefulness. Examples of the pharmaceutical additives include excipients, binders, disintegrants, lubricants, fluidizing agents (anti-caking agents), colorants, capsule coatings, coating agents, plasticizers, flavor-correcting agents, sweeteners, flavoring agents, solvents, solubilizing aids, emulsifiers, suspending agents (adhesives), thickeners, pH adjusters (acidifiers, alkalizers, buffers), wetting agents (solubilizers), antimicrobial preservatives, chelating agents, suppository bases, ointment bases, hardening agents, softeners, water for medical use, propellants, stabilizers, and preservatives. These pharmaceutical additives are readily selected by those skilled in the art according to the intended dosage form and route of administration, as well as standard pharmacological practices.

The therapeutic agent can be in any dosage form. Examples of the dosage form include eye drops, tablets, capsules, internal use, external use, suppositories, injections, and inhalations.

The therapeutic agent may be prescribed, if appropriate, at the discretion of the physician or health care professional. The dose and administration plan of the therapeutic agent may also be determined, if appropriate, at the discretion of the physician or health care professional.

The route of administration of the therapeutic agent is selected, if appropriate, according to factors such as the dosage form of the therapeutic agent and the type and severity of the disease to be treated. As examples of the administration route, Subretinal injection, Vitreous injection, and Suprachoroidal injection are preferably employed.

### Examples

### [Example 1: Identification of ZF target sequences]

### 1. Search for candidate sequences

Around a 100 bp region located upstream of the start codon of exon 1 of the RHO gene, possible sites of ZFN-mediated double-stranded DNA break were searched. The 100 bp region was selected as a region of low evolutionary sequence conservation.

ZFFinger Tools (https://www.scripps.edu/barbas/zfdesign/zfdesignhome.php), a publicly available tool from the Barbas Laboratory of the Scripps Research Institute, were used to search for candidate sequences to which ZFNs could bind. The binding target sequence of each zinc-finger array of ZFN dimers is 18 bp (consisting of 6 ZFs). When the length of nucleotides (spacer) between the binding target sequences was set to 6 bp or 5 bp, 34 pairs of candidate binding target sequences were provided (see Table 1).

The amino acid sequence of each ZF used the amino acid sequence described in the literature ("Standardized reagents and protocols for engineering zinc-finger nucleases by modular assembly", Wright et al., Nature Protocols, 2006, Vol. 1, No. 3, p. 1637-52, Supplemental Table 1). A zinc-finger array consisted of a total of 6 ZFs, each corresponding to a three-nucleotide sequence, at the 3' end side of the target sequence. For example, the sense strand ZFN 4350S, which has a zinc-finger array recognizing the nucleotide sequence of SEQ ID NO: 1 (ATTCTTGGGTGGGAGCAG), possesses an amino acid sequence in which ZF91, ZF62, ZF106, ZF58, ZF103, and ZF88 - which recognize CAG, GAG, TGG, GGG, CTT, and ATT, respectively (see Supplemental Table 1 in the above-mentioned literature) - are linked in this order from the N-terminus.

**[Table 1-1]**

| Pair NO: | | ZFN name | Recognition sequence | Recognition sequence SEQ ID NO: | ZF array amino acid sequence SEQ ID NO: |
|---|---|---|---|---|---|
| 1 | 4350S | Sense strand | 5'- ATT CTT CCC TGG GAG CAG -3' | 1 | 59 |
| | 4350AS | Antisense stand | 5'- AAG GCC TGA GCT CAG CCA -3' | 2 | 60 |
| 2 | 4653S | Sense strand | 5'- CTT GGG TGG GAG CAG CCA -3' | 3 | 61 |
| | 4653AS | Antisense stand | 5'- GCG AAG GCC TGA GCT CAG -3' | 4 | 62 |
| 3 | 4956S | Sense strand | 5'- GGG TGG GAG CAG CCA CGG -3' | 5 | 63 |
| | 4956AS | Antisense stand | 5'- GCT GCG AAG GCC TGA GCT -3' | 6 | 64 |
| 4 | 5158S | Sense strand | 5'- GTG GGA GCA GCC ACG GGT -3' | 7 | 65 |
| | 5158AS | Antisense stand | 5'- ATG CTG CGA AGG CCT CAG -3' | 8 | 66 |
| 5 | 5259S | Sense strand | 5'- TGG GAG CAG CCA CGG GTC -3' | 9 | 67 |
| | 5259AS | Antisense stand | 5'- AAT GCT GCG AAG GCC TGA -3' | 10 | 68 |
| 6 | 5461S | Sense strand | 5' - GGA GCA GCC ACG GGT CAG -3' | 11 | 69 |
| | 5461AS | Antisense stand | 5'- AGA ATG CTG CGA AGG CCT -3' | 12 | 70 |
| 7 | 5562S | Sense strand | 5'- GAG CAG CCA CGG GTC AGC -3' | 13 | 71 |
| | 5562AS | Antisense stand | 5'- AAG AAT GCT GCG AAG CCC -3' | 14 | 72 |
| 8 | 5764S | Sense strand | 5'- GCA GCC ACG GGT CAG CCA -3' | 15 | 73 |
| | 5764AS | Antisense stand | 5'- CCA AGA ATG CTG CGA AGG -3' | 16 | 74 |
| 9 | 5865S | Sense strand | 5'- CAG CCA CGG GTC AGC CAC -3' | 17 | 75 |
| | 5865AS | Antisense stand | 5'- CCC AAG AAT GCT GCG AAG -3' | 18 | 76 |
| 10 | 6067S | Sense strand | 5'- GCC ACG GGT CAG CCA CAA -3' | 19 | 77 |
| | 6067AS | Antisense stand | **5'- CAC CCA ACA ATG CTG** CGA -3' | 20 | 78 |
| 11 | 61685 | Sense strand | 5'- CCA CGG GTC AGC CAC AAG -3' | 21 | 79 |
| | 6168AS | Antisense stand | 5'- CCA CCC AAG AAT GCT GCG -3' | 22 | 80 |
| 12 | 7178S | Sense strand | 5'- GCC ACA AGG GCC ACA GCC -3' | 23 | 81 |
| | 7178AS | Antisense stand | 5'- GTG GCT GCT CCC ACC CAA -3' | 24 | 82 |
| 13 | 7481S | Sense strand | **5'- ACA AGG GCC ACA GCC ATG** -3' | 25 | 83 |
| | 7481AS | Antisense stand | 5'- CCC GTG GCT GCT CCC ACC -3' | 26 | 84 |
| 14 | 77845 | Sense strand | 5'- AGG GCC ACA GCC ATG AAT -3' | 27 | 85 |
| | 7784AS | Antisense stand | **5'- TGA CCC** GTG GCT GCT CCC -3' | 28 | 86 |
| 15 | 8087S | Sense strand | 5'- GCC ACA GCC ATG AAT GGC -3' | 29 | 87 |
| | 8087AS | Antisense stand | 5'- GGC TGA CCC GTG GCT GCT -3' | 30 | 88 |
| 16 | 8289S | Sense strand | 5'- CAC AGC CAT GAA TGG CAC -3' | 31 | 89 |
| | 8289AS | Antisense stand | 5'- GTG GCT GAC CCG TGG CTG -3' | 32 | 90 |
| 17 | 8592S | Sense strand | 5'- AGC CAT GAA TGG CAC AGA -3' | 33 | 91 |
| | 8592AS | Antisense stand | 5'- CTT GTG GCT GAC CCG TGG -3' | 34 | 92 |
| 18 | 8895S | Sense strand | 5'- CAT GAA TGG CAC AGA AGG -3' | 35 | 93 |
| | 9895AS | Antisense stand | 5'- GCC CTT GTG GCT GAC CCG -3' | 36 | 94 |
| 19 | 4955S | Sense strand | 5'- TGG GTG GGA GCA GCC ACG -3' | 37 | 95 |
| | 4956AS | Antisense strand | 5'- GCT GCG AAG GCC TGA GCT -3' | 6 | 64 |
| 20 | 5157S | Sense strand | 5'- GGT GGG AGC AGC CAC GGG -3' | 38 | 96 |
| | 5158AS | Antisense stand | 5'- ATG CTG CGA AGG CCT GAG -3' | 8 | 66 |
| 21 | 5158S | Sense strand | 5'- GTG GGA GCA GCC ACG GGT -3' | 7 | 65 |
| | 5259AS | Antisense stand | 5'- AAT GCT GCG AAG GCC TGA -3' | 10 | 68 |
| 22 | 5461S | Sense strand | 5'- GGA GCA GCC ACG GGT CAG -3' | 11 | 69 |
| | 5562AS | Antisense stand | 5'- AAG AAT GCT GCG AAG GCC -3' | 14 | 72 |
| 23 | 5764S | Sense strand | 5'- CCA GCC ACG GGT CAG CCA -3' | 15 | 73 |
| | 5865AS | Antisen se stand | **5'- CCC AAG AAT GCT GCG AAG** -3' | 18 | 76 |
| 24 | 6067S | Sense strand | 5'- GCC ACG GGT CAG CCA CAA -3' | 19 | 77 |
| | 6168AS | Antisense stand | 5'- CCA CCC AAG AAT GCT GCG -3' | 22 | 80 |
| 25 | 6874S | Sense strand | 5'- GTC AGC CAC AAG GGC CAC -3' | 39 | 97 |
| | 6874AS | Antisense stand | 5'- GCT GCT CCC ACC CAA GAA -3' | 40 | 98 |

**[Table 1-2]**

| | | | | | |
|---|---|---|---|---|---|
| 26 | 7177 S | Sense strand | 5'- AGC CAC AAG GGC CAC AGC -3' | 41 | 99 |
| | 7178AS | Antisense stand | 5'- GTG GCT GCT CCC ACC CAA -3' | 24 | 82 |
| 27 | 7480S | Sense strand | 5'- CAC AAG GGC CAC AGC CAT -3' | 42 | 100 |
| | 7481AS | Antisense strand | 5'- CCC GTG GCT GCT CCC ACC -3' | 26 | 84 |
| 28 | 7783S | Sense strand | 5'- AAG GGC CAC AGC CAT GAA -3' | 43 | 101 |
| | 7784AS | Antisense strand | 5'- TGA CCC GTG GCT GCT CCC -3' | 28 | 86 |
| 29 | 8086S | Sense strand | 5'- GGC CAC AGC CAT GAA TGG -3' | 44 | 102 |
| | 8087AS | Antisense stand | 5'- GGC TGA CCC GTG GCT GCT -3' | 30 | 88 |
| 30 | 8288S | Sense strand | 5'- CCA CAG CCA TGA ATG GCA -3' | 45 | 103 |
| | 8289AS | Antisense strand | 5'- GTG GCT GAC CCG TGG CTG -3' | 32 | 90 |
| 31 | 8591S | Sense strand | 5'- CAG CCA TGA ATG GCA CAG -3' | 46 | 104 |
| | 8592AS | Antisense strand | 5'- CTT GTG GCT GAC CCG TGG -3' | 34 | 92 |
| 32 | 88928 | Sense strand | 5'- CCA TGA ATG GCA CAG AAG -3' | 47 | 105 |
| | 8895AS | Antisense strand | 5'- GCC CTT GTG GCT GAC CCG -3' | 36 | 94 |
| 33 | 9197S | Sense strand | 5'- TGA ATG GCA CAG AAG GCC -3' | 48 | 106 |
| | 9197AS | Antisense strand | 5'- GTG GCC CTT GTG GCT GAC -3' | 49 | 107 |
| 34 | 9410S | Sense strand | 5'- ATG GCA CAG AAG GCC CTA -3' | 50 | 108 |
| | 9410AS | Antisense strand | 5'- GCT GTG GCC CTT GTG GCT -3' | 51 | 109 |

### 2. Evaluation of ZFN cleavage activity against candidate binding target sequences

The cleavage activity against each candidate binding target sequence was evaluated by SSA (Single strand annealing) analysis using the DDD-type variant (ND1DDD, SEQ ID NO: 57) and the RRR-type variant (ND1RRR, SEQ ID NO: 58) of ND1 as a nuclease.

The SSA analysis quantitatively evaluates the ZFN cleavage activity against the binding target sequences by using gene homologous recombination and single-strand annealing (SSA), which are gene repair mechanisms when intracellular DNA breaks occur. HEK293 cells and the following expression vectors were used for the analysis.

### (1) ZF-ND1 expression plasmids (pCMV-ZF-ND1DDD and pCMV-ZF-ND1RRR)

These plasmids express "ZF-ND1" under the control of the CMV promoter.

In the notation "ZF-ND1", "ZF" indicates one of the sense strand and antisense strand ZFNs of the 34 ZFN pairs listed in Table 1, and "ND1" indicates one of the subunits (ND1DDD and ND1RRR) of the ND1 heterodimer. ND1DDD was used for the sense strand ZFN, and ND1RRR was used for the antisense strand ZFN. A nuclear localization signal (NLS) was attached to the 5' side of "ZF", and "ZF" and "ND1" were linked using a linker sequence (Thr-Gly-Gly-Ser: SEQ ID NO: 56). The poly(A) sequence was added to the 3' side of "ND1". The poly(A) sequence of rabbit beta-globin was used for the poly(A) sequence.

### (2) EGFP fragment expression plasmid (pCAG-EGxxFP)

This plasmid expresses EGFP under the control of the CAG promoter.

EGxxFP contains a 1-600 bp fragment of the EGFP gene, a stop codon, a multicloning site, and a 120-720 bp fragment of the EGFP gene, and candidate binding target sequences are inserted into the multicloning site.

The pCAG-EGxxFP plasmid expresses incomplete GFP due to a stop codon immediately after the 1-600 bp fragment of the EGFP gene when no ZF-ND1-mediated cleavage occurred between the candidate binding target sequences. On the other hand, when ZF-ND1-mediated cleavage occurred between the candidate binding target sequences, gene homologous recombination and single-strand annealing (SSA) occur between overlapping sequences in the 120-720 bp fragment of the EGFP gene, resulting in expression of the complete EGFP gene and thus causing EGFP fluorescence.

pCMV-ZF-ND1DDD, pCMV-ZF-ND1RRR, and pCAG-EGxxFP were transfected into HEK293 cells according to the attached protocol using a transfection reagent (FuGene6 (Promega) or Viafect (Promega)). Fluorescent images were taken 48 hours after transfection. The results are shown in Fig. 1. EGFP green fluorescence was observed in 16 of the 34 pairs of candidate binding target sequences (1, 3-8, 16, 18-23, 30, and 32), confirming that ZF-ND1-mediated cleavage occurred between the binding target sequences. High fluorescence intensity was observed for pairs 3, 5, 6, 19, and 21-23, demonstrating especially high cleavage activity.

### [Example 2: Examination of high expression vector constructs]

ZFN high expression plasmid vectors were examined.

In general, there are methods to express two different genes: one method in which one promoter is used to express two different genes (bicistronic expression); and the other method in which two different promoters are used to express each gene.

In this Example, as an example of the former, a plasmid vector (hereinafter referred to as "Bicistronic") was constructed in which ZF-S-ND1DDD and ZF-AS-ND1RRR were linked via a self-cleaving peptide (Furin-P2A) under the control of the CMV promoter. Furthermore, as an example of the latter, a plasmid vector was constructed in which a CMV promoter and a human EF1α (Elongation Factor alpha-1) promoter were arranged in the same direction (hereinafter referred to as "Dual promoter") or in the opposite direction (hereinafter referred to as "Bidirectional promoter"). Fig. 2 shows the configuration of each plasmid vector. The Dual and Bidirectional promoters express ZF-S-ND1DDD under the control of EF1a promoter and ZF-AS-ND1RRR under the control of CMV promoter. For the target binding sequences of ZFs (ZF-S and ZF-AS), the pair No. 22 (5461S and 5562AS) in Table 1 was used. In the Dual promoter, an insulator sequence was placed to prevent interference between the two gene expressions.

SSA analysis was performed as in Example 1 while using Bicistronic, Dual promoter, and Bidirectional promoter plasmid vectors. As a positive control, the ZF-ND1 expression plasmids (positive control) using the pair No. 22 in Example 1 were used.

The results are shown in Fig. 3. For the Dual promoter, EGFP expression was hardly detected. In addition, EGFP signal was attenuated in Bicistronic compared to the positive control. By contrast, EGFP expression caused by the bidirectional promoter was equivalent to that in the positive control.

Using the Bicistronic and Bidirectional promoter plasmid vectors, the InDel (Insertion and Deletion) frequencies in the target RHO sequence were compared by Cel-I analysis. As a positive control, the ZF-ND1 expression plasmids (positive control) using the pair No. 22 in Example 1 was used. The Cel-I analysis was performed using the recommended protocol of the GeneArt Genomic Cleavage Detection kit (ThermoFisher Scientific) using genomic DNA from HEK293 cells transfected with each plasmid construct. The target RHO sequence amplified by PCR was treated with Cel-I nuclease, and the electrophoresis results are shown in Fig. 4. In addition, Fig. 5 shows the results of quantification by analysis site (http://shinyapps.datacurators.nl/tide/) of Tide (Tracking of Indels by DEcomposition) of electrophenogram obtained after the direct sequencing of PCR amplified products. Fig. 5 shows each InDel histogram where 0 on the X-axis indicates the same sequence as WT (no InDel), the positive direction indicates the number of Insertion nucleotides, the negative direction indicates the number of Deletion nucleotides, and the Y axis indicates their frequency (percentage). The "Total eff" indicates the sum (%) of InDel frequencies.

In genomic DNA of non-transfected cells, a band was identified at 345 bp (see lane 2 of Fig. 4). In the positive control, two bands (220 bp and 130 bp) due to cleavage by Cel-I nuclease (see lanes 3 and 5 of Fig. 4) were detected because InDel occurred, and the frequency of InDel was 25.7% (see Fig. 5). In the Bicistronic, the amount of cleaved DNA was reduced compared to the positive control (see lane 4 of Fig. 4), and the frequency of InDel was as low as 4.6% (see Fig. 5). By contrast, for the Bidirectional promoter, the same amount of cleaved DNA as of the positive control (see lane 6 of Fig. 4) and substantially the same frequency of InDel (see Fig. 5) were detected.

The results of this study indicate that the Bidirectional promoter enables two different genes to be integrated into one construct and is useful for industrial applications such as AAV gene therapy preparations.

### [Sequence listing free text]

SEQ ID NO. 1: recognition sequence of the zinc-finger array of ZFN 4350S
SEQ ID NO. 2: recognition sequence of the zinc-finger array of ZFN 4350AS
SEQ ID NO. 3: recognition sequence of the zinc-finger array of ZFN 4653S
SEQ ID NO. 4: recognition sequence of the zinc-finger array of ZFN 4653AS
SEQ ID NO. 5: recognition sequence of the zinc-finger array of ZFN 4956S
SEQ ID NO. 6: recognition sequence of the zinc-finger array of ZFN 4956AS
SEQ ID NO. 7: recognition sequence of the zinc-finger array of ZFN 5158S
SEQ ID NO. 8: recognition sequence of the zinc-finger array of ZFN 5158AS
SEQ ID NO. 9: recognition sequence of the zinc-finger array of ZFN 5259S
SEQ ID NO. 10: recognition sequence of the zinc-finger array of ZFN 5259AS
SEQ ID NO. 11: recognition sequence of the zinc-finger array of ZFN 5461S
SEQ ID NO. 12: recognition sequence of the zinc-finger array of ZFN 5461AS
SEQ ID NO. 13: recognition sequence of the zinc-finger array of ZFN 5562S
SEQ ID NO. 14: recognition sequence of the zinc-finger array of ZFN 5562AS
SEQ ID NO. 15: recognition sequence of the zinc-finger array of ZFN 5764S
SEQ ID NO. 16: recognition sequence of the zinc-finger array of ZFN 5764AS
SEQ ID NO. 17: recognition sequence of the zinc-finger array of ZFN 5865S
SEQ ID NO. 18: recognition sequence of the zinc-finger array of ZFN 5865AS
SEQ ID NO. 19: recognition sequence of the zinc-finger array of ZFN 6067S
SEQ ID NO. 20: recognition sequence of the zinc-finger array of ZFN 6067AS
SEQ ID NO. 21: recognition sequence of the zinc-finger array of ZFN 6168S
SEQ ID NO. 22: recognition sequence of the zinc-finger array of ZFN 6168AS
SEQ ID NO. 23: recognition sequence of the zinc-finger array of ZFN 7178S
SEQ ID NO. 24: recognition sequence of the zinc-finger array of ZFN 7178AS
SEQ ID NO. 25: recognition sequence of the zinc-finger array of ZFN 7481S
SEQ ID NO. 26: recognition sequence of the zinc-finger array of ZFN 7481AS
SEQ ID NO. 27: recognition sequence of the zinc-finger array of ZFN 7784S
SEQ ID NO. 28: recognition sequence of the zinc-finger array of ZFN 7784AS
SEQ ID NO. 29: recognition sequence of the zinc-finger array of ZFN 8087S
SEQ ID NO. 30: recognition sequence of the zinc-finger array of ZFN 8087AS
SEQ ID NO. 31: recognition sequence of the zinc-finger array of ZFN 8289S
SEQ ID NO. 32: recognition sequence of the zinc-finger array of ZFN 8289AS
SEQ ID NO. 33: recognition sequence of the zinc-finger array of ZFN 8592S
SEQ ID NO. 34: recognition sequence of the zinc-finger array of ZFN 8592AS
SEQ ID NO. 35: recognition sequence of the zinc-finger array of ZFN 8895S
SEQ ID NO. 36: recognition sequence of the zinc-finger array of ZFN 8895AS
SEQ ID NO. 37: recognition sequence of the zinc-finger array of ZFN 4955S
SEQ ID NO. 38: recognition sequence of the zinc-finger array of ZFN 5157S
SEQ ID NO. 39: recognition sequence of the zinc-finger array of ZFN 6874S
SEQ ID NO. 40: recognition sequence of the zinc-finger array of ZFN 6874AS
SEQ ID NO. 41: recognition sequence of the zinc-finger array of ZFN 7177S
SEQ ID NO. 42: recognition sequence of the zinc-finger array of ZFN 7480S
SEQ ID NO. 43: recognition sequence of the zinc-finger array of ZFN 7783S
SEQ ID NO. 44: recognition sequence of the zinc-finger array of ZFN 8086S
SEQ ID NO. 45: recognition sequence of the zinc-finger array of ZFN 8288S
SEQ ID NO. 46: recognition sequence of the zinc-finger array of ZFN 8591S
SEQ ID NO. 47: recognition sequence of the zinc-finger array of ZFN 8892S
SEQ ID NO. 48: recognition sequence of the zinc-finger array of ZFN 9197S
SEQ ID NO. 49: recognition sequence of the zinc-finger array of ZFN 9197AS
SEQ ID NO. 50: recognition sequence of the zinc-finger array of ZFN 9410S
SEQ ID NO. 51: recognition sequence of the zinc-finger array of ZFN 9410AS
SEQ ID NO. 52: amino acid sequence of the full-length protein (from *Bacillus* sp. SGD-V-76) containing ND1
SEQ ID NO. 53: nucleotide sequence of the full-length protein (from *Bacillus* sp. SGD-V-76) containing ND1
SEQ ID NO. 54: amino acid sequence of the full-length protein (from *Clostridium botulinum*) containing ND2
SEQ ID NO. 55: nucleotide sequence of the full-length protein (from *Clostridium botulinum*) containing ND2
SEQ ID NO. 56: amino acid sequence of a peptide linker
SEQ ID NO. 57: amino acid sequence of ND1DDD
SEQ ID NO. 58: amino acid sequence of ND1RRR
SEQ ID NO. 59: amino acid sequence of ZFN 4350S
SEQ ID NO. 60: amino acid sequence of ZFN 4350AS
SEQ ID NO. 61: amino acid sequence of ZFN 4653S
SEQ ID NO. 62: amino acid sequence of ZFN 4653AS
SEQ ID NO. 63: amino acid sequence of ZFN 4956S
SEQ ID NO. 64: amino acid sequence of ZFN 4956AS
SEQ ID NO. 65: amino acid sequence of ZFN 5158S
SEQ ID NO. 66: amino acid sequence of ZFN 5158AS
SEQ ID NO. 67: amino acid sequence of ZFN 5259S
SEQ ID NO. 68: amino acid sequence of ZFN 5259AS
SEQ ID NO. 69: amino acid sequence of ZFN 5461S
SEQ ID NO. 70: amino acid sequence of ZFN 5461AS
SEQ ID NO. 71: amino acid sequence of ZFN 5562S
SEQ ID NO. 72: amino acid sequence of ZFN 5562AS
SEQ ID NO. 73: amino acid sequence of ZFN 5764S
SEQ ID NO. 74: amino acid sequence of ZFN 5764AS
SEQ ID NO. 75: amino acid sequence of ZFN 5865S
SEQ ID NO. 76: amino acid sequence of ZFN 5865AS
SEQ ID NO. 77: amino acid sequence of ZFN 6067S
SEQ ID NO. 78: amino acid sequence of ZFN 6067AS
SEQ ID NO. 79: amino acid sequence of ZFN 6168S
SEQ ID NO. 80: amino acid sequence of ZFN 6168AS
SEQ ID NO. 81: amino acid sequence of ZFN 7178S
SEQ ID NO. 82: amino acid sequence of ZFN 7178AS
SEQ ID NO. 83: amino acid sequence of ZFN 7481S
SEQ ID NO. 84: amino acid sequence of ZFN 7481AS
SEQ ID NO. 85: amino acid sequence of ZFN 7784S
SEQ ID NO. 86: amino acid sequence of ZFN 7784AS
SEQ ID NO. 87: amino acid sequence of ZFN 8087S
SEQ ID NO. 88: amino acid sequence of ZFN 8087AS
SEQ ID NO. 89: amino acid sequence of ZFN 8289S
SEQ ID NO. 90: amino acid sequence of ZFN 8289AS
SEQ ID NO. 91: amino acid sequence of ZFN 8592S
SEQ ID NO. 92: amino acid sequence of ZFN 8592AS
SEQ ID NO. 93: amino acid sequence of ZFN 8895S
SEQ ID NO. 94: amino acid sequence of ZFN 8895AS
SEQ ID NO. 95: amino acid sequence of ZFN 4955S
SEQ ID NO. 96: amino acid sequence of ZFN 5157S
SEQ ID NO. 97: amino acid sequence of ZFN 6874S
SEQ ID NO. 98: amino acid sequence of ZFN 6874AS
SEQ ID NO. 99: amino acid sequence of ZFN 7177S
SEQ ID NO. 100: amino acid sequence of ZFN 7480S
SEQ ID NO. 101: amino acid sequence of ZFN 7783S
SEQ ID NO. 102: amino acid sequence of ZFN 8086S
SEQ ID NO. 103: amino acid sequence of ZFN 8288S
SEQ ID NO. 104: amino acid sequence of ZFN 8591S
SEQ ID NO. 105: amino acid sequence of ZFN 8892S
SEQ ID NO. 106: amino acid sequence of ZFN 9197S
SEQ ID NO. 107: amino acid sequence of ZFN 9197AS
SEQ ID NO. 108: amino acid sequence of ZFN 9410S
SEQ ID NO. 109: amino acid sequence of ZFN 9410AS

## Claims

1. A combination of a pair of zinc-finger nucleases (ZFNs) for genome editing of a rhodopsin (RHO) gene locus, the combination selected from the following group:
(1) a combination of a ZFN with a zinc-finger array recognizing a nucleotide sequence of SEQ ID NO: 1 and a ZFN with a zinc-finger array recognizing a nucleotide sequence of SEQ ID NO: 2;
(2) a combination of a ZFN with a zinc-finger array recognizing a nucleotide sequence of SEQ ID NO: 5 and a ZFN with a zinc-finger array recognizing a nucleotide sequence of SEQ ID NO: 6;
(3) a combination of a ZFN with a zinc-finger array recognizing a nucleotide sequence of SEQ ID NO: 7 and a ZFN with a zinc-finger array recognizing a nucleotide sequence of SEQ ID NO: 8;
(4) a combination of a ZFN with a zinc-finger array recognizing a nucleotide sequence of SEQ ID NO: 9 and a ZFN with a zinc-finger array recognizing a nucleotide sequence of SEQ ID NO: 10;
(5) a combination of a ZFN with a zinc-finger array recognizing a nucleotide sequence of SEQ ID NO: 11 and a ZFN with a zinc-finger array recognizing a nucleotide sequence of SEQ ID NO: 12;
(6) a combination of a ZFN with a zinc-finger array recognizing a nucleotide sequence of SEQ ID NO: 13 and a ZFN with a zinc-finger array recognizing a nucleotide sequence of SEQ ID NO: 14;
(7) a combination of a ZFN with a zinc-finger array recognizing a nucleotide sequence of SEQ ID NO: 15 and a ZFN with a zinc-finger array recognizing a nucleotide sequence of SEQ ID NO: 16;
(8) a combination of a ZFN with a zinc-finger array recognizing a nucleotide sequence of SEQ ID NO: 31 and a ZFN with a zinc-finger array recognizing a nucleotide sequence of SEQ ID NO: 32;
(9) a combination of a ZFN with a zinc-finger array recognizing a nucleotide sequence of SEQ ID NO: 35 and a ZFN with a zinc-finger array recognizing a nucleotide sequence of SEQ ID NO: 36;
(10) a combination of a ZFN with a zinc-finger array recognizing a nucleotide sequence of SEQ ID NO: 37 and a ZFN with a zinc-finger array recognizing a nucleotide sequence of SEQ ID NO: 6;
(11) a combination of a ZFN with a zinc-finger array recognizing a nucleotide sequence of SEQ ID NO: 38 and a ZFN with a zinc-finger array recognizing a nucleotide sequence of SEQ ID NO: 8;
(12) a combination of a ZFN with a zinc-finger array recognizing a nucleotide sequence of SEQ ID NO: 7 and a ZFN with a zinc-finger array recognizing a nucleotide sequence of SEQ ID NO: 10;
(13) a combination of a ZFN with a zinc-finger array recognizing a nucleotide sequence of SEQ ID NO: 11 and a ZFN with a zinc-finger array recognizing a nucleotide sequence of SEQ ID NO: 14;
(14) a combination of a ZFN with a zinc-finger array recognizing a nucleotide sequence of SEQ ID NO: 15 and a ZFN with a zinc-finger array recognizing a nucleotide sequence of SEQ ID NO: 18;
(15) a combination of a ZFN with a zinc-finger array recognizing a nucleotide sequence of SEQ ID NO: 45 and a ZFN with a zinc-finger array recognizing a nucleotide sequence of SEQ ID NO: 32; and
(16) a combination of a ZFN with a zinc-finger array recognizing a nucleotide sequence of SEQ ID NO: 47 and a ZFN with a zinc-finger array recognizing a nucleotide sequence of SEQ ID NO: 36.

2. The combination according to claim 1, wherein a nuclease domain of each ZFN is ND1.

3. A vector comprising a nucleotide sequence encoding a pair of ZFNs according to claim 1.

4. The vector according to claim 3, wherein the vector is a bidirectional expression vector and
the vector comprises a nucleotide sequence encoding a sense strand ZFN and a promoter sequence controlling transcription of the nucleotide sequence, and
a nucleotide sequence encoding an antisense strand ZFN and a promoter sequence controlling transcription of the nucleotide sequence,
provided that the two promoter sequences are arranged consecutively without a spacer sequence therebetween.

5. A therapeutic agent for a disease derived from an RHO gene, comprising the vector according to claim 3 or 4.

6. A method for genome editing of an RHO gene locus, comprising, as a procedure, introducing the vector according to claim 3 or 4 into a photoreceptor cell.

7. A method for producing a photoreceptor cell having an RHO gene genome-edited, comprising, as a procedure, introducing the vector according to claim 3 or 4 into a photoreceptor cell.
